# NEW EUROPEAN PATENT SPECIFICATION

(11) **EP 0 295 248 B2**
(45) Date of publication and mention of the opposition decision: **28.04.1999**
(45) Mention of the grant of the patent: 31.03.1993
(21) Application number: 87901176.5
(22) Date of filing: 13.01.1987
(51) Int. Cl.: B01J 13/02, A61K 9/52

(54) **LIPOSOME PREPARATION AND ANTIBIOTIC**
LIPOSOMES PRÄPARAT UND ANTIBIOTIKUM
PREPARATION ANTIBIOTIQUE A BASE DE LIPOSOMES

(30) Priority: 23.12.1986 US 946391; 23.12.1986 US 946398
(43) Date of publication of application: 21.12.1988
(60) Divisional application: 92106330.1
(73) Proprietor: THE LIPOSOME COMPANY, INC., Princeton, NJ 08540 (US)
(72) Inventor: BALLY, Marcel, B., Vancouver, V6T1M8 (CA); BOLCSAK, Lois, E., Lawrenceville, NJ 08648 (US); CULLIS, Pieter, R., Vancouver, V6J3R2 (CA); JANOFF, Andrew, S., Yardley, PA 19067 (US); MAYER, Lawrence, D., Vancouver, V6K2A5 (CA); LENK, Robert, P., Lambertville, NJ 08530 (US); JEDRUSIAK, Jo, Ann, Robbinsville, NJ 08691 (US)
(74) Representative: Warcoin, Jacques
(86) International application number: US8700077
(87) International publication number: WO8804573

(56) References cited:
- WO-A-85/00515
- WO-A-85/00751
- WO-A-87/00043
- JP-A- 6 080 767
- US-A- 3 993 754
- US-A- 4 016 100
- US-A- 4 145 410
- US-A- 4 224 179
- US-A- 4 235 871
- US-A- 4 397 846
- US-A- 4 515 736
- US-A- 4 522 803
- US-A- 4 532 089
- US-A- 4 588 578
- US-A- 4 610 868
- US-A- 6 362 995
- CHEMICAL ABSTRACTS, vol. 93, no. 4, 28th July 1980, page 326, abstract no. 31732q, Columbus, Ohio, US; J.R. MORGAN et al.: "Preparation and properties of liposome-associated gentamicin"
- CHEMICAL ABSTRACTS, vol. 101, no. 2, 9th July 1984, page 294, abstract no. 12083w, Columbus, Ohio, US; M.A. VLADIMIRSKII et al.: "Liposome preparations of streptomycin and dihydrostreptomycin"
- CHEMICAL ABSTRACTS, vol. 103, no. 20, 18th November 1985, page 368, abstract no. 166095e, Columbus, Ohio, US; M.W. FOUNTAIN et al.: "Treatment of Brucella canis and Brucella abortus in vitro and in vivo by stable plurilamellar vesicle-encapsulated aminoglycosides"
- Current microbiology (1981), 6, pp. 373-376
- Biochimica et Biophysica Acta (1980), 596, pp. 420-425
- Veterinary Immunology and Immunopathology (1985), 8, pp. 171-182
- Investigative Ophtalmology and visual science (1984), 25, pp. 486-490
- Investigative Ophthalmology and visual science (1986), 27, pp. 1103-1106
- Biochemical pharmacology (1986), 35, pp. 3761-3765
- The Merck Index, 10th ed (1983), pp. 60, 627, 758, 925, 926, 1010, 1011, 1185, 1186, 1226, 1227 and 1358
- Textbook of Pharmacology (Bowman and Rand), 2nd ed., 1980, Table 34.1

## Description

### FIELD OF THE INVENTION

This invention relates to aminoglycosides, analogs and derivatives thereof, and salts as well as the process for making and utilizing same. nonguanidino aminoglycoside liposomes, their preparation and use, are particularly described. Nonguanidino nonphosphate aminoglycoside liposomes with drug to lipid ratios of greater than 3:25 (eq. wt.) are disclosed.

EP-A-231261 (WO 87/00043) is comprised in the state of the Art under art 54(3) and (4) and discloses the incorporation of tobramycin phosphate in FATMLVs.

### BACKGROUND OF THE INVENTION

Aminoglycosides are a class of compounds characterized by the ability to interfere with protein synthesis in micro-organisms. Aminoglycosides consist of two or more amino sugars joined in a glycoside linkage to a hexose (or aminocyclitol) nucleus. The hexose nuclei thus far known are either streptidine or 2-deoxystreptamine, though others may be anticipated. Aminoglycoside families are distinguished by the amino sugar attached to the aminocyclitol. For example, the neomycin family comprises three amino sugars attached to the central 2-deoxystreptamine. The kanamycin and glutamicin families have only two amino sugars attached to the aminocyclitol.

Aminoglycosides include: neomycin B. paromomycin, ribostamycin, lividomycin, kanamycin A,, kanamycin B, amikacin, tobramycin, viomycin, gentamicin C₁, gentamicin C₁ₐ, (gentamicin C₂, C₁, C₁ₐ and analogs and derivatives thereof collectively "gentamicin"), sisomicin, netilmicin, streptomycin and dihydrostreptomycin. Streptomycin and dihydrostreptomycin characterized by the presence of a guanadino group are understood to be unique in associating with liposomes in higher drug to lipid ratios than the nonguanidino aminoglycosides. The term "nonguanidino" aminoglycosides will include aminoglycosides other than aminoglycosides bearing a guanidino group.

Unfortunately, use of these compounds has been limited by several factors. Often directed to use in preventing protein synthesis in bacteria, bacteria have demonstrated a remarkable capacity to resist the inhibitory effect of aminoglycosides. Resistance of an organism to aminoglycoside action occurs with a broad range of aminoglycosides. A further problem of aminoglycoside use has been characteristically poor gastric absorption and rapid excretion. Injection of aminoglycosides results in rapid peak plasma concentration often in the neighborhood of 30 to 90 minutes following intramuscular injection which is associated with toxicity. Another limitation is that the aminoglycosides fail to enter the CNS or the eye.

In the therapeutic use of aminoglycosides in animals, including humans, serious problems of toxicity have been noted. For example, therapeutic use in higher animals may be accompanied by ototoxicity potentially involving both auditory and vestibular functions as well as nephrotoxicity, and neuromuscular blockade culminating in respiratory distress.

It is an object of this invention to provide for aminoglycosides with improved liposomal association. It is a further object of this invention to provide a method of manufacture of liposomes associated with aminoglycoside. It is another object of this invention that said liposomes substantially associate with said aminoglycoside. It is an additional object of this invention that the liposomes of this invention provide a high aminoglycoside to lipid ratio particularly as to nonguanidino aminoglysocides. It is a further object of this invention to provide such liposomes in a pharmaceutical dosage form for therapeutic treatment of an animal including a human.

It is another object of this invention to provide an intravenously administrable form of aminoglycoside without concommitant immediate availability of unbound or unassociated aminoglycoside at high plasma levels.

### SUMMARY OF THE INVENTION

It has now been discovered that nonguanidino nonphosphate aminoglycosides, analogs and derivatives thereof, in the form of sulfate and other salts, have surprisingly useful therapeutic properties. Aminoglycoside salts are found to be particuarly adapted to association with liposomes. The term aminoglycoside will be understood to include analogs and derivatives thereof.

In the past nonguandino aminoglycosides were found to be in rather limited association with liposomes. For example, Morgan et al. "Preparation and Properties of Liposome-Associated Gentamicin" Antimicrobial Agents and Chemotherapy, 17:544-548 (1980) reports about 4 mg of gentamicin or less associating with 100 mg of lipid. In the present invention liposomes are "associated" with enhanced levels of a nonguanidino aminoglycoside frequently at least about 40% of available aminoglycoside. This is true for both guanidino and nonguanidino aminoglycosides. The term "associated" shall be understood to be immobilized on or in a liposome, within the aqueous phase of a liposome or within the lipid phase of a liposome.

The enhanced association of lipid to nonguanadino aminoglycoside has further enabled the production of nonguanidino nonphosphate aminoglycoside liposomes at 3:25 ratio (w/w) eq. wt. in a preferred embodiment, a ratio over 1:5 (w/w) greater than 20 mg (actual weight) of nonguanidino aminoglycoside (sulfate salt) per 100 mg of lipid. Weights of aminoglycoside may be expressed as actual weight of the aminoglycoside (actual weight "act.wt.") or the equivalent weight or base equivalent of the drug molecule not including the weight of the counterion (equivalent weight "eq. wt."). The process of this invention permits the preparation of aminoglycoside liposomes and preparations containing such liposomes to be manufactured in a wide range of potencies higher than obtained without this process.

It is a particular advantage of this invention that enhanced drug to lipid ratio preparations require reduced administration of lipid per drug dosage thus avoiding or reducing toxicity associated with lipid administration.

The enhanced association of available drug with liposomes in the preparation of the liposomes reduces the need for drug and lipid starting materials.

Finally, high potency pharmaceutical preparations are consequently of smaller volume and thus cause less tissue insult upon administration. This is particularly true as to intramuscular administration.

Methods of preparing and utilizing aminoglycoside liposomes by the modified SPLV process of, in the most preferred embodiment, requiring both drying liposomes to powder and stabilizing liposomes, are also described more fully below.

This invention deals with SPLV vesicles associated with aminoglycosides such as neomycin B, paromomycin, ribostamycin, lividomycin, kanamycin A, kanamycin B, amikacin, tobramycin, gentamicin C₁, gentamicin C₁ₐ, gentamicin C₂, netilmicin, and sisomicin and phospholipids such as phosphatidylinositol, phosphatidylcholine, phosphatidylethanolamine, phosphatidylserine and phosphatidylglycerol alone or in combination with other lipids.

The liposomes of this invention include SPLV liposomes comprising at least one lipid and at least one nonguanidino aminoglycoside, preferably in the form of a sulfate salt. Further included are SPLV liposomes associated with aminoglycosides such as neomycin B, paromomycin, ribostamycin, lividomycin, kanamycin A, kanamycin B, amikacin, tobramycin, gentamicin C₁. gentamicin C₁ₐ, gentamicin C₂, netilmicin, and sisomicin and phospholipids such as phosphatidylinositol, phosphatidylcholine, phosphatidylethanolamine, pnosphatiaylserine and phosphatidylglycerol. This invention includes the methods of making aminoglycoside associated liposomes and particularly aminoglycoside sulfate associated liposomes as described below, and particularly the liposomes substantially associating with available aminoglycoside sulfate. Also included are nonguanidino aminoglycoside liposomes of greater than 12.0 mg and preferably greater than 10 mg of nonguanidino aminoglycoside per 100 mg of lipid, (eq. weight) corresponding to drug to lipid ratios of 3:25 and 5:25 respectively.

This invention includes liposomes comprising at least one nonguanidino aminoglycoside present in at least 12.4 mg eq. wt. nonguanidino aminoglycoside per 100 mg lipid. This further includes such nonguanidino aminoglycosides as neomycin B, paromomycin, ribostamycin, lividomycin. kanamycin A,, kanamycin B, amikacin, tobramycin, viomycin, gentamicin C₁, gentamicin C₁ₐ (gentamicin C₂, C₁, C₁ₐ analogs and derivatives thereof collectively "gentamicin"), sisomicin, netilmicin, and preferably nonguanidino aminoglycosides in the form of sulfate salts.

This invention includes liposomes comprising at least one amphipathic lipid. This invention includes the process of enhancing aminoglycoside to lipid drug ratios in liposomes of the SPLV Process by variously drying the lipid-organic solvent-aminoglycoside-aqueous phase mixture to powder, permitting the rehydrated powder to stabilize by standing at 4°C for at least 8 hours and preferably for one day. This invention further includes having dried the lipid-organic solvent-aminoglycoside-aqueous phase mixture over a three to eight hour Period and preferably a five hour period. Alternatively, the lipid-organic solvent-aminoglycoside-aqueous phase may be dryed only to a slurry or paste.

This invention further includes removal of liposomally unassociated aminoglycoside from aminoglycoside liposome preparation by low energy sepratory methods such as dialysis or chromotography being careful to avoid high energy sepratory methods such as centrifugation.

### DETAILED DESCRIPTION OF THE INVENTION

The utility of aminoglycoside containing liposomes is described in connection with the treatment of disease in animals in U.S. Patent No. 4,552,803 to Lenk et al.

For nonguanidino aminoglycoside by substantially associated it is to be understood that no more than about 60% of the nonguanadino aminoglycoside present in a liposomal preparation remains free in solution hence unassociated with the liposomes.

Liposomes are vesicles comprising closed bilayer membranes containing an entrapped aqueous phase. Liposomes of the invention are SPLV.

The liposomes of this invention may be prepared so as to associate with nonguanidino aminoglycoside in ratios equal to or greater than 12 mg aminoglycoside per 100 mg lipid and as high as 30 mg nonguanidino aminoglycoside per 100 mg of lipid or higher.

The original liposome preparation of Bangham et al. (1965, J. Mol. Biol. 13:238-252) involves suspending phospholipids in an organic solvent which is then evaporated to dryness leaving a phospholipid film on the reaction vessel. Then an appropriate amount of aqueous phase is added, the mixture is allowed to "swell", and the resulting liposomes which consist of multilamellar vesicles (hereinafter referred to as MLVs) are dispersed by mechanical means. The structure of the resulting membrane bilayer is such that the hydrophobic (nonpolar) "tails" of the lipid orient toward the center of the bilayer while the hydrophilic (polar) "heads" orient towards the aqueous phase.

In the practice of this invention a class of liposomes characterized as having substantially equal interlamellar solute distribution is preferred. This class of liposomes is denominated as stable plurilamellar vesicles (SPLV) as defined in U.S. Patent No. 4,522,803 to Lenk et. al.

The term lipid as used herein shall mean any suitable material resulting in a bilayer such that a hydrophobic portion of the lipid material orients toward the bilayer while a hydrophilic portion orients toward the aqueous phase.

Two general classes of lipid compounds are useful in the present invention. The most prominent members are highly hydrophobic compounds, such as triglycerides. Corn oil serves as a convenient and economical source of mixed triglycerides, but other vegetable oils, including but not limited to palm kernel oil, coconut oil, soybean oil, sunflower oil, safflower oil and cocoa butter and may be used. Specific molecular species might be employed as well. Such species may include, but are not limited to, trilaurin, trimyristin, tripalmitin and tristearin, or other glyceryl esters in which the fatty acyl chains of these compounds as well as other fatty acids are incorporated in a non-homogeneous fashion. Other broad classes of long chain hydrophobic compounds such as the wide range of cholesterol esters may be used. It has even been found that long chain organic mixtures such as petroleum jelly are acceptable lipid materials.

Further a variety of cholesterols and other sterols and their water soluble derivatives have been used to form aminoglcoside liposomes; see specifically Janoff et al., U.S. Patent Application Serial No. 773,429, filed September 10, 1985 entitled "Steroidal Liposomes". Mayhew et al., WO 85/00968, published March 14, 1985, describes a method for reducing the toxicity of drugs by encapsulating them in liposomes comprising alpha-tocopherol and certain derivatives thereof. Also, a variety of tocopherols and their water soluble derivatives have been used to form liposomes, see Janoff et al., U.S. Patent Application Serial No. 786,740, filed October 15, 1986 entitled "Alpha-Tocopherol- Based Vesicles". Preferred of this group are cholesterol hemisuccinate and tocopherol hemisuccinate. The only constraint appears to be that the hydrophobic compounds selected should, when uncomplexed with the other components of this invention, be soluble in a particular organic solvent chosen for use in the manufacture of the liposomes.

The second broad class of lipid materials used in this invention as to aminoglycoside are amphipathic in character. Hydrophilic character could be imparted to the molecule through the presence of phosphato, carboxylic, sulphato, amino, sulfhydryl and nitro groups. Hydrophobicity could be conferred by the inclusion of groups that include, but are not limited to, long chain saturated and unsaturated aliphatic hydrocarbon groups and such groups substituted by one or more aromatic, cycloaliphatic or heterocyclic group. The preferred amphipathic compounds are phosphoglycerides, representative examples of which include phosphatidylcholine, phosphatidylethanolamine, lysophosphatidylcholine, lysophosphatidylethanolamine, phosphatidylserine, phosphatidylinositol, phosphatidic acid, dimyristoylphosphatidylglycerol and diphosphatidylglycerol alone or in combination with other lipids. Synthetic saturated compounds such as dimyristoylphosphatidylcholine, dipolmitoylphosphatidylcholine, or distearoylphosphatidylcholine or unsaturated species such as dioleoylphosphatidylcholine or dilinoleoylphosphatidylcholine might also be usable. Other compounds lacking phosphorous, such as members of the sphingolipid and glycosphingolipid families, are also within the group designated as lipid.

Amphipathic lipids are necessary as the primary liposomal structural element for aminoglycosides made by the modified SPLV preparation described below. In forming modified SPLV preparation aminoglycoside liposomes, these ampnipathic lipids may be admixed with other lipids including triglycerides and sterols.

A method for preparing the sterol containing liposomes involves adding to an aqueous buffer a salt form of an organic acid derivative of a sterol capable of forming closed bilayers in an amount sufficient to form completely closed bilayers which entrap an aqueous compartment. A suspension of multilamellar vesicles is formed by shaking the mixture. The formation of vesicles is facilitated if the aqueous buffer also contains the counterion of the salt in solution.

Liposomes entrap an aqueous medium which is enclosed by the lipid bilayers. The aqueous medium can be for example, water or water containing a dissolved salt or buffer. Examples of such salts or buffers can be sodium chloride and phosphate buffered saline (PBS). Other buffers include but are not limited to borate, citrate, Tris-HCI (Tris-(hydroxymethyl)-aminomethane hydrochloride), and HEPES (N-2-hydroxyethyl piperazine-N'-2-ethane sulfonic acid). Buffers may be in the pH range of between 2.0 and 14.0.

In a liposome-drug delivery system, the therapeutic agent, here aminoglycoside, is entrapped in the liposome and then administered to the patient to be treated. For example, see Rahman et al., U.S. Patent No. 3,993,754: Sears, U.S. Patent No. 4,145,410; Papahadjopoulos et al., U.S. Patent No. 4,235,871; Schneider. U.S. Patent No. 4,224,179. Lenk, et al., U.S. Patent No. 4,522,803. and Fountain et al., U.S. Patent No. 4,588,578.

Pharmaceutical liposomal preparations are, in the preferred embodiment, delivered in physiological saline or water buffered with phosphate or citrate appropriate for injection.

Optionally, the aminoglycoside liposomes can be dehydrated, thereby enabling storage for extended periods of time until use. Standard freeze-drying equipment or equivalent apparatus may be used to dehydrate the liposomes. Liposomes may also be dehydrated simply by placing them under reduced pressure. Alternatively, the liposomes and their surrounding medium can be frozen in liquid nitrogen prior to dehydration. Dehydration with prior freezing may be performed in the presence of one or more protective sugars in the preparation, according to the process of Janoff et al., U.S. Patent Application Serial No. 759,419, filed July 26, 1985, entitled "Dehydrated Liposomes". Examples of protective sugars that may be used include, but are not limited to, trehalose, maltose, sucrose, glucose, lactose and dextran. Alternatively, multilamellar vesicles may be dehydrated with prior freezing without protective sugars. When the dehydrated liposomes are to be used, rehydration is accomplished by methods which include simply adding an aqueous solution, e.g., distilled water, to the liposomes and allowing them to rehydrate.

The aminoglycosides of this invention are administered associated with liposomes, and if desired, in admixture with a pharmaceutically-acceptable carrier (such as physiological saline or phosphate buffer selected with regard to the intended route of administration and standard pharmaceutical practice. Dosages for aminoglycosides when associated with liposomes will often be about that of the aminoglycoside alone; dosages will be set by the prescribing medical professional considering many factors including the age. weight and condition of the patient. The ratio of active ingredient to carrier will naturally depend on the chemical nature, solubility and stability of the aminoglycoside, as well as the dosage contemplated. For parenteral administration or injection via such routes as intravenous, intraperitoneal, intramuscular, subcutaneous, or intra-mammary route, sterile solutions of the liposome composition are prepared. For intravenous use, the total concentration of solutes should be controlled to render the preparation isotonic.

In another example of their use, liposomal associated aminoglycosides may be incorporated into a broad range of topical dosage forms including but not limited to gels, oils, emulsions and the like. For instance, the suspension containing the liposomal associated aminoglycoside may be added to the aqueous phase as an ingredient in the liposome preparation. Such preparations may be administered as topical creams, pastes, ointments, gels, lotions and the like for direct application.

### Pharmacological Use of Aminoglycosides

The aminoglycosides may be classified as broad spectrum antibiotics. With respect to antibacterial spectrum, there are similarities among them, but there are also considerable differences, hence generalizations should be avoided. The aminoglycosides of this invention and liposomally associated aminoglycosides are useful in therapeutically-effective doses in the treatment of gram-negative pneumonia.

Aminoglycosides may be administered in combination with one or more pharmaceutically acceptable carriers. Such carriers are well known in the art. Aminoglycosides are preferably administered intramuscularly or intravenously. Ophthalmic solutions and ointments are also available for topical ophthalmic applications. Creams are available in some cases for topical application.

Gentamicin (base equivalent) for example, may be administered IM or IV at about 1 to 1.7 mg/kg of body weight about every eight hours or 0.75 to 1.25 mg/kg every six hours for about seven to ten days. However, many considerations are involved in determing an actual dosage including incidence of renal failure, the aminoglycoside in use, the animal and its presenting condition. A therapeutically effective dose of an aminoglycoside will be that dosage which, in view of the specifics of the application, produce the desired result.

### Modified SPLV Preparation of Aminoglycoside Enhanced Drug to Lipid Ratio Liposomes

SPLVs produced by a novel method are prepared as follows: An amphipathic lipid or mixture of lipids is dissolved in an organic solvent. This constitutes the first mixture. Many organic solvents are suitable, but diethyl ether, halogenated hydrocarbons and mixtures of halogenated hydrocarbons and ether are preferred with methylene chloride most preferred. To this solution are added an aqueous phase and the aminoglycoside to be liposomally associated. Aminoglycoside sulfate is most preferred at this stage. This biphasic mixture is converted to SPLVs by emulsifying the aqueous material within the organic solvent while evaporating the solvent and in the preferred embodiment evaporating to dryness. Evaporation can be accomplished by any evaporative technique, e.g., evaporation by passing a stream of inert gas over the mixture, by heating, or by vacuum. Drying, and particularly drying to powder, in the preferred embodiment is accomplished over 3 to 8 hours and preferably over 5 hours and preferably while stirring and most preferably stirring at high speed. Drying temperature is between 25°C to 45°C. The temperature must not be at or above the boiling point of the solvent in use which of course will vary with the pressure of the system. With methylene chloride 40°C is preferred. If not drying to powder, than drying to a paste or slurry is acceptable. The volume of organic solvent used must be proportionate to the aqueous volume so that the aqueous material can be completely emulsified in the mixture. In practice, a minimum of roughly 1 volume of solvent to 1 volume of aqueous phase may be used. In fact the ratio of solvent to aqueous phase can vary to up to 100 or more volumes of solvent to 1 volume aqueous phase. The amount of lipid must be sufficient so as to exceed that amount needed to coat the emulsion droplets (about 40 mg of lipid per ml of aqueous phase). The upper boundary is limited only by the practicality and efficiency, but SPLVs can be made with 15 gm of lipid per ml of aqueous phase.

If a powder has been formed, the resulting preparation is then rehydrated. It is a limitation of this process that the material be permitted to "stabilize" for a period of time. The temperature for stabilizing is not critical but colder temperatures (above freezing) enjoy less liposomal degradation. Thus stabilizing as performed preferably at 4°C. The period of stabilization is at minimum 8 hours and preferably at least one day. After stabilizing unassociated aminoglycoside may be removed, which is done in the preferred embodiment.

The removal of unassociated aminoglycoside must be done in a manner that does not adversely affect liposomal integerity. Dialysing against a saline solution is such a low energy type non-adverse process. Centrifugation is a high energy process that could adversely affect liposomal integrity and should not be utilized. Other low energy type processes known in the art such as chromatography may also be used.

Prior to the removal of unassociated aminoglycoside, the liposomes may be sized. If for intravenous use in humans these are preferably sized to 3 to 5 um - the size beyond which capillary blockage can occur.

Sizing can be performed by any method including homogenization and extrusion such as by steel mesh, straight path or tortuous path filtration, including membrane filters of polycarbonate and other polymeric substances.

Optimal results require that the liposome mixture be dried to powder prior to rehydration. A further requirement for optimal results is that the liposomes stand to stabilize overnight prior to removal of unassociated aminoglycoside. Further the most preferred results by this process arise from utilizing the sulfate salt form of aminoglycoside. This is particularly true of gentamicin. However, aminoglycoside salts including chloride, and tartrate are contemplated as well as aminoglycoside free base. Aminoglycoside salts formed with hydrophobic moieties are also contemplated within this invention. Such hydrophobic moieties are fatty acids, for example, palmitate, myristate, and stearate.

By the foregoing method of preparing liposomes of this invention the aminoglycoside is associated with liposomes at enhanced levels does not remain free in solution. By substantially associated it is to be understood that no more than 60% of the nonguanidino aminoglycoside present in a preparation is not associated with the liposomes.

In some instances, liposome dispersions having a nonguanidino nonphosphate aminoglycoside concentration of greater than 200 mg (eq. wt.) per gm of EPC were obtained and in some instances as high as 360 mg (eq. wt.) were obtained.

Aminoglycoside associated liposomes and particularly nonphosphate aminoglycosides of this invention made by the modified SPLV preparation have enhanced drug to lipid ratios compared to previous techniques. The liposomes of this invention may be prepared so as to associate with nonguanidino aminoglycoside in ratios 3:25 which is 12.0 mg nonguanadino nonphosphate aminoglycoside (eq. wt.) per 100 mg of lipid or higher. This results in a more concentrated and thus potent nonguanadino aminoglycoside liposome preparation.

### Example 1

### Comparative Liposomal Entrapment of Nonguanidino Aminoglycoside Sulfate and Aminoglcoside Phosphate By Standard Procedure

Amikacin A comparative test of the liposomal association of amikacin-SO₄ and amikacin-PO₄ at pH 2.0 was performed. To form the sulfate, the free base of amikacin in phosphate buffered saline was titrated to pH 2.0 with H₂SO₄. To form the phosphate, amikacin in phosphate buffered saline was titrated to pH 2.0 with 85% H₃PO₄ (weight/volume). Amikacin association was compared at 10, 37, 50 and 80 mg. Liposomes were then prepared by the SPLV method of Lenk et al. U.S. Patent No. 4,522,803. Amikacin/liposomal association was determined by spectrophotometric assay and was in each instance at least about 1/3 greater for the amikacin phosphate salt form than for the amikacin sulfate form.

### Example 2

### Modified SPLV Preparation of Aminoglycoside Enhanced Drug to Lipid Ratio Liposomes

### Preparation of Precursor Liposomes

One g of lipid (egg phosphatidyl choline "EPC") was dried to a film in 500 ml round bottom flask. The lipid was resuspended using about 50 ml of methylene chloride.

An aqueous solution of aminoglycoside was added to the mixture. The aqueous solution was 0.5 gm gentamicin sulfate (act. wt.) in 9 ml of 0.9% saline (weight/volume). The resulting mixture was agitated by stirring. This process created precursor liposomes.

### Drying, Rehydration, and Standing Process

The liposomal mixture was stirred under nitrogen atmosphere. The pressure was reduced but maintained below boiling until the sample was dried to a powder. This process took about 4 to 5 hours at 40°C. The mixture was rehydrated with about 9 ml of distilled water and 41 ml of 0.9% saline (weight/volume) under nitrogen. The mixture was stirred until a milky color was achieved with no aggregates or clumps of material. This procedure took 2 hours at about 40°C. The mixture was left standing or stabilizing at 4°C for one day.

### Dialyzing

The sample was then sized to about 3um by extrusion at pressures of up to about 4268 kPa (700 psi) through a polycarbonate straight path membrane filter. After filtration the material was dialyzed about 2 days against 0.9% saline (weight/volume). The dialysis removed substantially all aminoglycoside not liposomally associated. The results of this process are shown in Tables 1 and 2. Table 1 shows the enhanced efficiency of association of the lipid with the aminoglycoside by this procedure versus not drying the liposomes to powder and not dialyzing but centrifuging off unassociated aminoglycoside.

Table 2 shows the enhanced aminoglycoside to lipid ratio attained by the use of the procedure versus not drying the liposomes to powder and not dialyzing but centrifuging off the unassociated aminoglycoside.

**Table 1**

| Comparative Efficiency of Liposomal Association of Gentamicin Sulfate % of Association of Available Gentamicin | |
|---|---|
| First mixture not dried to powder | First mixture dried to powder |
| Unassociated gentamicin removed by Centrifuge | Unassociated gentamicin removed by Dialysis |
| 27.03 | 35.3 |
| 24.58 | 72.6 |
| 17.27 | 51.2 |
| 22.02 | 62.0 |
| 32.71 | 41.0 |

**Table 2**

| Comparative Amount of Gentamicin Sulfate Liposomal Association in mg Drug/mg EPC (eq. wt.) mg Gentamicin/100 mg lipid | |
|---|---|
| First mixture is not dried to powder | First mixture is dried to powder |
| Unassociated gentamicin is is removed by Centrifuge | Unassociated gentamicin removed by Dialysis |
| 12.5 | 17.5 |
| 11.5 | 36.1 |
| 11.3 | 13.0 |
| 8.4 | 23.1 |
| 10.8 | 20.5 |

## Claims (Claims for the following Contracting State(s): BE, CH, DE, FR, GB, IT, LI, LU, NL, SE)

1. Liposomes comprising at least one nonguanidino nonphosphate aminoglycoside present in a drug:lipid ratio (eq.wt.) of at least 3:25 (w/w), wherein the liposomes are SPLV liposomes.

2. The liposomes of claim 1 wherein the aminoglycoside is neomycin B, paromomycin, ribostamycin, lividomycin, kanamycin A, kanamycin B, amikacin, tobramycin, gentamicin, gentamicin C₁, gentamicin C₁ₐ, gentamicin C₂, netilmicin, or sisomicin.

3. The liposomes of any one of claims 1 or 2 wherein the aminoglycoside is in the form of a sulfate salt.

4. The liposomes of claim 3, wherein the aminoglycoside is gentamicin sulfate.

5. The liposomes of any one of claims 1 to 4 wherein the liposomes are comprised of at least one amphipathic lipid.

6. A pharmaceutical composition comprising a therapeutically effective amount of liposomes in accordance with any one of claims 1 to 5.

7. The pharmaceutical composition of claim 6 for use in the treatment of gram-negative pneumonia.

8. A method of preparing SPLV liposomes in accordance with any of claims 1 to 5, said method comprising the steps of:
a) dissolving at least one amphipathic lipid in organic solvent;
b) adding to the solution formed by step a) an aqueous phase and an aminoglycoside, thus forming a biphasic mixture ;
c) emulsifying the aqueous phase within the organic solvent while evaporating the solvent ;
d) stabilizing the material in a hydrated state for at least 8 hours.

9. The method of claim 8 further comprising removing aminoglycoside not liposomally associated after completion of step d).

10. The method of claim 8 or 9 wherein the evaporating of step c) includes evaporating the material to dryness and rehydration prior to step d).

11. The method of any one of claims 8 to 10 wherein the stabilizing time of step d) is at least 24 hours.

## Claims (Claims for the following Contracting State(s): AT)

1. Process for the preparation of SPLV liposomes comprising at least one nonguanidino nonphosphate aminoglycoside, in which the process comprises associating the aminoglycoside with the liposome in a drug to lipid ratio (eq.wt.) of at least 3:25 (w/w).

2. The process of claim 1 wherein the aminoglycoside is neomycin B, paromomycin, ribostamycin, lividomycin, kanamycin A, kanamycin B, amikacin, tobramycin, gentamicin, gentamicin C₁, gentamicin C₁ₐ, gentamicin C₂, netilmicin, or sisomicin.

3. The process of any one of claims 1 or 2 wherein the aminoglycoside is in the form of a sulfate salt.

4. The process of claim 3, wherein the aminoglycoside is gentamicin sulfate.

5. The process of any one of claims 1 to 4 wherein the liposomes are comprised of at least one amphipathic lipid.

6. The process of any of claims 1 to 5 comprising the steps of:
a) dissolving at least one amphipathic lipid in organic solvent;
b) adding to the solution formed by step a) an aqueous phase and an aminoglycoside, thus forming a biphasic mixture;
c) emulsifying the aqueous phase within the organic solvent while evaporating the solvent ;
d) stabilizing the material resulting from c) by maintaining the material in a hydrated state for at least 8 hours.

7. The process of claim 6 wherein the evaporating of step c) includes evaporating the material to dryness and rehydrating prior to step d).

8. The process of claims 6 or 7 wherein the stabilizing time of step d) is at least 24 hours.

9. The process according to anyone of claims 1 to 8 further comprising removing aminoglycoside not liposomally associated.

## Patentansprüche (Patentansprüche für folgende(n) Vertragsstaat(en): BE, CH, DE, FR, GB, IT, LI, LU, NL, SE)

1. Liposomen, umfassend wenigstens ein Nichtguanidin-Nichtphosphat-Aminoglycosid in einem Arzneimittel-zu-Lipid-Verhältnis (Äqu.gew.) von wenigstens 3:50 (w/w), worin die Liposomen SPLV-Liposomen sind.

2. Liposomen nach Anspruch 1, worin das Aminoglycosid Neomycin B, Paromomycin, Ribostamycin, Lividomycin, Kanamycin A, Kanamycin B, Amikacin, Tobramycin, Gentamicin, Gentamicin C₁, Gentamicin C₁ₐ, Gentamicin C₂, Netilmicin oder Sisomicin ist.

3. Liposomen nach einem der Ansprüche 1 oder 2, worin das Aminoglycosid in Form eines Sulfatsalzes vorliegt.

4. Liposomen nach Anspruch 3, worin das Aminoglycosid Gentamicinsulfat ist.

5. Liposomen nach einem der Ansprüche 1 bis 4, worin die Liposomen wenigstens ein amphipatisches Lipid umfassen.

6. Pharmazeutische Zusammensetzung, die eine therapeutisch wirksame Menge der Liposomen nach einem der Ansprüche 1 bis 5 enthält.

7. Pharmazeutische Zusammensetzung von Anspruch 6 zur Verwendung bei der Behandlung von gram-negativer Pneumonie.

8. Verfahren zur Herstellung von SPVL-Liposomen nach einem der Ansprüche 1 bis 5, wobei das Verfahren die Stufen umfaßt:
(a) Lösen von wenigstens einem amphipatischen Lipid in organischem Lösungsmittel;
(b) Zugabe einer wäßrigen Phase und eines Aminoglycosides zu der in Stufe (a) gebildeten Lösung, um so ein zweiphasiges Gemisch zu bilden;
(c) Emulgieren der wäßrigen Phase in dem organischen Lösungsmittel während des Verdampfens des Lösungsmittels;
(d) Stabilisieren des Materials in hydratisiertem Zustand über wenigstens 8 Stunden.

9. Verfahren nach Anspruch 8, weiterhin umfassend die Entfernung von Aminoglycosid, das nach Vollendung der Stufe (d) nicht liposomal assoziiert ist.

10. Verfahren nach Anspruch 8 oder 9, worin das Verdampfen von Stufe (c) das Verdampfen des Materials bis zur Trockne und das Re-Hydratisieren vor Stufe (d) mit einschließt.

11. Verfahren nach einem der Ansprüche 8 bis 10, worin die Stabilisierungszeit von Stufe (d) wenigstens 24 Stunden beträgt.

## Patentansprüche (Patentansprüche für folgende(n) Vertragsstaat(en): AT)

1. Verfahren zur Herstellung von SPLV-Liposomen, umfassend wenigstens ein Nichtguanidin-Nichtphosphat-Aminoglycosid, wobei das Verfahren das Assoziieren des Aminoglycosides mit dem Liposom in einem Arzneimittel-zu-Lipid-Verhältnis (Äqu.gew.) von wenigstens 3:50 (w/w) umfaßt.

2. Verfahren nach Anspruch 1, worin das Aminoglycosid Neomycin B, Paromomycin, Ribostamycin, Lividomycin, Kanamycin A, Kanamycin B, Amikacin, Tobramycin, Gentamicin, Gentamicin C₁, Gentamicin C₁ₐ, Gentamicin C₂, Netilmicin oder Sisomicin ist.

3. Verfahren nach einem der Ansprüche 1 oder 2, worin das Aminoglycosid in Form eines Sulfatsalzes vorliegt.

4. Verfahren nach Anspruch 3 , worin das Aminoglycosid Gentamicinsulfat ist.

5. Verfahren nach einem der Ansprüche 1 bis 4, worin die Liposomen wenigstens ein amphipatisches Lipid umfassen.

6. Verfahren nach einem der Ansprüche 1 bis 5, umfassend die Stufen:
(a) Lösen von wenigstens einem amphipatischen Lipid in organischem Lösungsmittel;
(b) Zugabe einer wäßrigen Phase und eines Aminoglycosides zu der in Stufe (a) gebildeten Lösung, um so ein zweiphasiges Gemisch zu bilden;
(c) Emulgieren der wäßrigen Phase in dem organischen Lösungsmittel während des Verdampfens des Lösungsmittels;
(d) Stabilisieren des aus (c) sich ergebenden Materials durch Halten des Materials in hydratisiertem Zustand über wenigstens 8 Stunden.

7. Verfahren nach Anspruch 6, worin das Verdampfen von Stufe (c) das Verdampfen des Materials bis zur Trockne und das Re-Hydrtisieren vor Stufe (d) mit einschließt.

8. Verfahren nach Anspruch 6 oder 7, worin die Stabilisierungszeit von Stufe (d) wenigstens 24 Stunden beträgt.

9. Verfahren nach einem der Ansprüche 1 bis 8, weiterhin umfassend die Entfernung des nicht liposomal assoziierten Aminoglycosides.

## Revendications (Revendications pour l'(les) Etat(s) contractant(s) suivant(s): BE, CH, DE, FR, GB, IT, LI, LU, NL, SE)

1. Liposomes comprenant au moins un aminoglycoside non guanidine, non phosphate, présent dans un rapport médicament/lipide (poids équivalent) d'au moins 3:25 (en poids/ poids), lesdits liposomes étant des SPLV.

2. Liposomes selon la revendication 1, dans lesquels l'aminoglycoside est la néomycine B, la paromomycine, la ribostamycine, la lividomycine, la kanamycine A, la kanamycine B, l'amikacine, la tobramycine, la gentamicine, la gentamicine C₁, la gentamicine C₁ₐ, la gentamicine C₂, la nétilmicine ou la sisomicine,

3. Liposomes selon l'une quelconque des revendications 1 ou 2, dans lesquels l'aminoglycoside est sous forme de sel sulfate.

4. Liposomes selon la revendication 3, dans lesquels l'aminoglycoside est le sulfate de gentamicine.

5. Liposomes selon l'une quelconque des revendications 1 à 4, dans lesquels les liposomes sont constitués d'au moins un lipide amphipathique.

6. Composition pharmaceutique comprenant une quantité efficace en thérapeutique de liposomes selon l'une quelconque des revendications 1 à 5.

7. Composition pharmaceutique selon la revendication 6, utilisable dans le traitement de la pneumonie Gram-négatif.

8. Procédé de préparation de liposomes SPLV selon l'une quelconque des revendications 1 à 5, ledit procédé comprenant les étapes qui consistent à :
(a) dissoudre au moins un lipide amphipathique dans un solvant organique;
(b) ajouter à la solution formée par l'étape (a) une phase aqueuse et un aminoglycoside, pour former un mélange à deux phases;
(c) émulsionner la phase aqueuse avec le solvant organique tout en évaporant le solvant;
(d) stabiliser le produit dans un état hydraté pendant au moins 8 heures.

9. Procédé selon la revendication 8, comprenant en outre l'élimination de l'aminoglycoside non associé à la manière d'un liposome, après l'achèvement de l'étape (d).

10. Procédé selon la revendication 8 ou 9, dans lequel l'évaporation de l'étape (c) comporte l'évaporation à sec du produit et sa réhydratation avant l'étape (d).

11. Procédé selon l'une quelconque des revendications 8 à 10, dans lequel la durée de stabilisation de l'étape (d) est d'au moins 24 heures.

## Revendications (Revendications pour l'(les) Etat(s) contractant(s) suivant(s): AT)

1. Procédé pour la préparation de liposomes SPLV comprenant au moins un aminoglycoside non guanidine, non phosphate, dans lequel le procédé comprend le fait d'associer l'aminoglycoside avec le liposome dans un rapport médicament/lipide (poids équivalent) d'au moins 3:25 (en poids/ poids).

2. Procédé selon la revendication 1, dans lequel l'aminoglycoside est la néomycine B, la paromomycine, la ribostamycine, la lividomycine, la kanamycine A, la kanamycine B, l'amikacine, la tobramycine, la gentamicine, la gentamicine C₁, la gentamicine C₁ₐ, la gentamicine C₂, la nétilmicine ou la sisomicine.

3. Procédé selon l'une quelconque des revendications 1 ou 2, dans lequel l'aminoglycoside est sous forme de sel sulfate.

4. Procédé selon la revendication 3, dans lequel l'aminoglycoside est le sulfate de gentamicine.

5. Procédé selon l'une quelconque des revendications 1 à 4, dans lequel les liposomes sont constitués d'au moins un lipide amphipathique.

6. Procédé selon l'une quelconque des revendications 1 à 5, ledit procédé comprenant les étapes qui consistent à :
(a) dissoudre au moins un lipide amphipathique dans un solvant organique;
(b) ajouter à la solution formée par l'étape (a) une phase aqueuse et un aminoglycoside, pour former un mélange à deux phases;
(c) émulsionner la phase aqueuse avec le solvant organique tout en évaporant le solvant;
(d) stabiliser le produit résultant de (c) en maintenant le produit dans un état hydraté pendant au moins 8 heures.

7. Procédé selon la revendication 6, dans lequel l'évaporation de l'étape (c) comporte l'évaporation à sec du produit et sa réhydratation avant l'étape (d).

8. Procédé selon les revendications 6 ou 7, dans lequel la durée de stabilisation de l'étape(d) est d'au moins 24 heures.

9. Procédé selon l'une quelconque des revendications 1 à 8, comprenant en outre l'élimination de l'aminoglycoside non associé aux liposomes.
